# EUROPEAN PATENT SPECIFICATION

(11) **EP 4 174 460 B1**
(45) Date of publication and mention of the grant of the patent: **27.05.2026**
(21) Application number: 21841388.8
(22) Date of filing: 08.07.2021
(51) Int. Cl.: G01J 5/00, G01K 13/00, G01J 5/02, G01J 5/08, G01J 5/80

(54) **BODY TEMPERATURE MEASUREMENT METHOD, ELECTRONIC DEVICE, AND STORAGE MEDIUM**
KÖRPERTEMPERATURMESSVERFAHREN, ELEKTRONISCHE VORRICHTUNG UND SPEICHERMEDIUM
PROCÉDÉ DE MESURE DE TEMPÉRATURE CORPORELLE, DISPOSITIF ÉLECTRONIQUE ET SUPPORT D'ENREGISTREMENT

(30) Priority: 14.07.2020 CN 202010673228
(43) Date of publication of application: 03.05.2023
(73) Proprietor: Huawei Technologies Co., Ltd., Shenzhen, Guangdong 518129 (CN)
(72) Inventor: WU, Xiaofang, Shenzhen, Guangdong 518129 (CN)
(74) Representative: Maiwald GmbH
(86) International application number: PCT/CN2021/105130
(87) International publication number: WO 2022/012401

(56) References cited:
- WO-A1-2019/015390
- CN-A- 105 125 181
- CN-A- 107 361 748
- CN-A- 111 310 692
- CN-A- 111 310 692
- CN-A- 111 323 145
- CN-U- 207 197 674
- JP-A- 2010 230 392
- JP-A- 2014 018 334
- US-A1- 2017 143 211
- US-A1- 2020 256 734

## Description

### TECHNICAL FIELD

Embodiments of this disclosure relate to the technical field of smart terminals, and in particular, to a body temperature measurement method, an electronic device, and a storage medium.

### BACKGROUND

People usually encounter body temperature measurement scenarios in daily life. For example, when a family member in a family has a fever, a thermometer may be used for measurement. A common thermometer is usually of a contact type, and is used to measure a value of an axillary temperature or an oral temperature of a user. However, objects using the contact-type thermometer are limited. For example, it is unsafe and inconvenient for children to use.

As technologies continuously develop, application scenarios of body temperature measurement are no longer limited to humans. For example, a temperature of a fed pet and a temperature of an object may be measured. For example, a temperature of water when milk powder is mixed may be measured. However, different types of objects, such as humans and animals, use different thermometers. In addition, it is very inconvenient to take along different types of thermometers.

WO 2019/015390 A1 discloses a body temperature measurement method and device, the method comprising: upon receiving a temperature measurement instruction, turning on a camera and a first infrared thermometer; photographing a user with the camera so as to obtain an image, performing identification on the image so as to determine a part of the user's body to perform temperature measurement on; acquiring a distance between the first infrared thermometer and the user; if the distance is consistent with a preset distance threshold, controlling the first infrared thermometer to measure the temperature of the part of the user's body to be measured, wherein the preset distance threshold is configured according to a focal length of a Fresnel lens of the first infrared thermometer; determining a body temperature or a body temperature range for the user according to a preset rule and a temperature obtained by measurement.
CN 111310692 A discloses a detection object management method. The detection object management method comprises the steps of obtaining multiple types of images of a detection object; determining a target detection area of the detection object based on the multiple types of images; acquiring a detection index of the target detection area; compensating the detection index according to the attribute of the target detection area; and judging whether the detection object is a target object or not according to the compensated detection index.
US 2017/143211 A1 discloses a device having a health detecting function, a display apparatus, a system and a health detecting method are provided, capable of monitoring physical condition and discovering an illness in time. The device having the health detecting function includes a processing module and a storage module. The processing module is configured for receiving image information of a detected person and storing the image information in the storage module.

### SUMMARY

Embodiments of this disclosure provide a body temperature measurement method, an electronic device, and a storage medium, to provide a manner that can be used to measure body temperatures of various types of objects, so that a user can measure the body temperatures of the different types of objects, and accuracy of body temperature measurement can be increased.

According to a first aspect of the invention, a body temperature measurement method with the steps defined in claim 1 is provided.

In a possible implementation, the method further includes:
obtaining a first distance to the target object, and comparing the first distance with a preset distance threshold; and if the first distance does not match the preset distance threshold, displaying a first prompt; or if the first distance matches the preset distance threshold, displaying a second prompt, where specifically, the first distance may be a distance between an infrared sensor for measuring a temperature and the target object; and because the temperature is measured by using the infrared sensor with a value of an optimum distance, the optimum distance may be obtained by measuring the first distance.

In a possible implementation, the method further includes:
associating the second temperature value with an identity of the target object, and storing the second temperature value, where specifically, the stored second temperature value may be used as a historical temperature value, where the historical temperature value may be bound with the target object as a personal historical database.

In a possible implementation, the method further includes:
performing facial recognition on the target object, to obtain the identity of the target object; performing querying according to the identity of the target object, to obtain a historical temperature value of the target object; and comparing the second temperature value with the historical temperature value, to determine, based on a comparison result, whether the second temperature value is normal.

In a possible implementation, the determining, based on the first feature, whether the second temperature value is normal includes:
performing querying based on the first feature, to obtain a preset temperature threshold corresponding to the first feature, where specifically, the preset temperature threshold may be a specific value or a value range, and this is not limited in this embodiment of this disclosure; and
comparing the second temperature value with the preset temperature threshold, and determining, based on a comparison result, whether the second temperature value is normal.

In a possible implementation, the method further includes:
obtaining a first image, and displaying the second temperature value on the first image, where specifically, the first image may be any photo, where the photo may be a currently captured photo or a photo pre-stored in a local album, and this is not limited in this embodiment of this disclosure.

A further aspect of the disclosure provides a body temperature measurement apparatus, including:
a recognition module, configured to detect a first operation for selecting a temperature measurement mode, determine a current temperature measurement mode in response to the detected first operation, and recognize a target object in the current temperature measurement mode to obtain a first feature of the target object;
a temperature measurement module, configured to detect a second operation for measuring a temperature of the target object, and measure the temperature of the target object in response to the detected second operation, to obtain a first temperature value;
a calibration module, configured to obtain a second feature, and compensate for the first temperature value based on the second feature, to obtain a second temperature value; and
a display module, configured to determine, based on the first feature, whether the second temperature value is normal, and display the second temperature value and a prompt indicating whether the second temperature value is normal.

In a possible implementation, the apparatus further includes:
a distance measurement module, configured to: obtain a first distance to the target object, and compare the first distance with a preset distance threshold; and if the first distance does not match the preset distance threshold, display a first prompt; or if the first distance matches the preset distance threshold, display a second prompt.

In a possible implementation, the apparatus further includes:
a storage module, configured to associate the second temperature value with an identity of the target object, and store the second temperature value.

In a possible implementation, the apparatus further includes:
a facial recognition module, configured to: perform facial recognition on the target object, to obtain the identity of the target object; perform querying according to the identity of the target object, to obtain a historical temperature value of the target object; and compare the second temperature value with the historical temperature value, to determine, based on a comparison result, whether the second temperature value is normal.

In a possible implementation, the display module includes:
a querying unit, configured to perform querying based on the first feature, to obtain a preset temperature threshold corresponding to the first feature; and
a comparison unit, configured to compare the second temperature value with the preset temperature threshold, and determine, based on a comparison result, whether the second temperature value is normal.

In a possible implementation, the apparatus further includes:
an association module, configured to obtain a first image, and display the second temperature value on the first image.

According to a further aspect of the invention, an electronic device with the features of claim 7 is provided.

In a possible implementation, when the foregoing instructions are executed by the foregoing device, the foregoing device is enabled to further perform the following steps:
obtaining a first distance to the target object, and comparing the first distance with a preset distance threshold; and
if the first distance does not match the preset distance threshold, displaying a first prompt; or if the first distance matches the preset distance threshold, displaying a second prompt.

In a possible implementation, when the foregoing instructions are executed by the foregoing device, the foregoing device is enabled to further perform the following steps:
associating the second temperature value with an identity of the target object, and storing the second temperature value.

In a possible implementation, when the foregoing instructions are executed by the foregoing device, the foregoing device is enabled to further perform the following steps:
performing facial recognition on the target object, to obtain the identity of the target object;
performing querying according to the identity of the target object, to obtain a historical temperature value of the target object; and
comparing the second temperature value with the historical temperature value, to determine, based on a comparison result, whether the second temperature value is normal.

In a possible implementation, when the foregoing instructions are executed by the foregoing device, the foregoing device is enabled to perform the determining, based on the first feature, whether the second temperature value is normal, including:
performing querying based on the first feature, to obtain a preset temperature threshold corresponding to the first feature; and
comparing the second temperature value with the preset temperature threshold, and determining, based on a comparison result, whether the second temperature value is normal.

In a possible implementation, when the foregoing instructions are executed by the foregoing device, the foregoing device is enabled to further perform the following steps:
obtaining a first image, and displaying the second temperature value on the first image.

According to a further aspect of the invention, a computer-readable storage medium with the features of claim 13 is provided. is run on a computer, the computer is enabled to perform the method in the first aspect.

According to a further aspect of the invention, a computer program product with the features of claim 14 is provided.

In a possible implementation, some or all of the programs may be stored in a storage medium packaged together with a processor, or may be stored in a memory not packaged together with a processor.

### BRIEF DESCRIPTION OF DRAWINGS

FIG. 1 is a schematic diagram of a structure of an electronic device according to an embodiment of this disclosure;
FIG. 2 is a schematic flowchart of a body temperature measurement method according to an embodiment of this disclosure;
FIG. 3 is a schematic diagram of a display interface for selecting a temperature measurement mode according to an embodiment of this disclosure;
FIG. 4 is a schematic diagram of a display interface during temperature measurement according to an embodiment of this disclosure;
FIG. 5 is a schematic diagram of a temperature measurement result display interface according to an embodiment of this disclosure; and
FIG. 6 is a schematic diagram of a structure of a body temperature measurement apparatus according to an embodiment of this disclosure.

### DESCRIPTION OF EMBODIMENTS

The following describes technical solutions in embodiments of this disclosure with reference to the accompanying drawings in embodiments of this disclosure. In the descriptions of embodiments of this disclosure, "/" means "or" unless otherwise specified. For example, A/B may represent A or B. In this specification, "and/or" describes only an association relationship for describing associated objects and represents that three relationships may exist. For example, A and/or B may represent the following three cases: Only A exists, both A and B exist, and only B exists.

The terms "first" and "second" mentioned below are merely intended for a purpose of description, and shall not be understood as an indication or implication of relative importance or implicit indication of the number of indicated technical features. Therefore, a feature limited by "first " or "second" may explicitly indicate or implicitly include one or more such features. In the description of the embodiment of this disclosure, unless otherwise stated, "multiple" means two or more than two.

Currently, common thermometers are usually contact-type thermometers. Different types of objects require different types of thermometers, such as a mercury thermometer, an ear thermometer, a pet thermometer, and an object thermometer. However, it is relatively troublesome and inconvenient for a user to take along a plurality of thermometers. In addition, the contact-type thermometers are also unsafe for children.

As technologies develop, infrared thermometers come into being. In nature, all objects with a temperature higher than absolute zero are constantly emitting infrared radiation energy to a surrounding space. However, a value of infrared radiation energy of an object and wavelength distribution of the object are very closely related to a surface temperature of the object. Therefore, the infrared energy radiated by the object is measured, so that the surface temperature of the object can be accurately measured and determined. In practical applications, the infrared thermometer usually measures the surface temperature of the object by using an infrared sensor. The infrared sensor is a sensor that performs data processing by using infrared rays, and has advantages of high sensitivity and the like. The infrared sensor can provide signals that are used to control and drive operation of an apparatus. In addition, the infrared sensor is usually used for non-contact temperature measurement. For example, the infrared sensor may be used to remotely measure a thermogram of a surface temperature of a human body, and a part with an abnormal temperature can be found.

However, due to different emissivity, different objects radiate different infrared energy with a same temperature. For example, infrared energy radiated by human skin and a wall surface both at 37 degrees centigrade is very different. Therefore, temperature values obtained by a same infrared thermometer measuring two objects with a same temperature may be different. In addition, an environment in which the object is located also affects a temperature value obtained through measurement, thereby causing a deviation between an actual temperature value and a measurement value.

Based on the foregoing problem, an embodiment of this disclosure provides a body temperature measurement method. Based on the foregoing method, accuracy of a measurement result can be increased.

A technical solution provided in this embodiment of this disclosure can be applied to an electronic device. The electronic device may be a mobile terminal, for example, a mobile phone. Alternatively, the electronic device may be a hand-held thermometer. A specific form of the electronic device for performing the technical solution is not particularly limited in this disclosure.

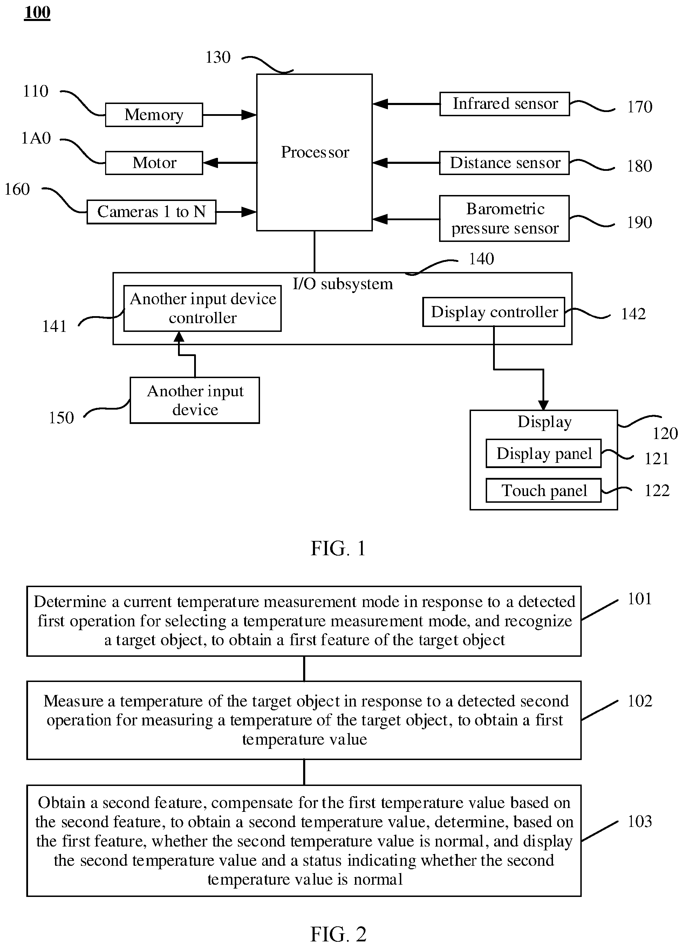

FIG. 1 is a schematic diagram of a structure of an electronic device 100 according to an embodiment of this disclosure. The electronic device 100 may include a memory 110, a display 120, a processor 130, an I/O subsystem 140, another input device 150, a camera 160, an infrared sensor 170, a distance sensor 180, a barometric pressure sensor 190, and a motor 1A0. The display 120 is configured to display a picture and an operation interface.

The display 120 may be configured to display information input by or provided for a user and various menus of the electronic device 100, and may further receive a user input. Specifically, the display 120 may include a display panel 121 and a touch panel 122. The display panel 121 may be configured in a form of a liquid crystal display (LCD, Liquid Crystal Display), an organic light-emitting diode (OLED, Organic Light-Emitting Diode), or the like. The touch panel 122, also referred to as a touchscreen, a touch-sensitive screen, or the like, can collect a contact operation or a non-contact operation (for example, an operation performed by the user on or near the touch panel 122 by using any suitable object or accessory such as a finger or a stylus; and the operation may also include a somatosensory operation that includes operations such as a single-point control operation and a multi-point control operation) of the user on or near the touch panel 122, and drive a corresponding connection apparatus based on a preset program. Optionally, the touch panel 122 may include two parts: a touch detection apparatus and a touch controller. The touch detection apparatus detects a touch orientation and a touch posture of the user, detects a signal brought by the touch operation, and transmits the signal to the touch controller. The touch controller receives touch information from the touch detection apparatus, converts the touch information into information that can be processed by the processor, then sends the information to the processor 130, and can receive and execute a command sent by the processor 130. In addition, the touch panel 122 may be implemented in various types, such as a resistive type, a capacitive type, an infrared type, and a surface acoustic wave type, or may be implemented by using any technology developed in the future. Further, the touch panel 122 may cover the display panel 121, and the user may perform, based on content displayed on the display panel 121 (the displayed content includes but is not limited to a soft keyboard, a virtual mouse, a virtual key, and an icon), an operation on or near the touch panel 122 covering the display panel 121. After detecting the operation on or near the touch panel 122, the touch panel 122 transmits the operation to the processor 130 by using the I/O subsystem 140, to determine a user input. Then the processor 130 provides a corresponding visual output on the display panel 121 based on the user input by using the I/O subsystem 140. Although in FIG. 1, the touch panel 122 and the display panel 121 act as two independent components to implement input and output functions of the electronic device 100, in some embodiments, the touch panel 122 and the display panel 121 may be integrated to implement the input and output functions of the electronic device 100.

The processor 130 is a control center of the electronic device 100, connects various parts of the entire electronic device by using various interfaces and lines, and executes various functions of the electronic device 100 and processes data by running or executing a software program and/or a module stored in the memory 110, and by invoking data stored in the memory 110, to monitor the entire electronic device 100. Optionally, the processor 130 may include one or more processing units. Preferably, the processor 130 may integrate an application processor and a modem processor. The application processor mainly processes an operating system, a user interface, an application, and the like. The modem processor mainly processes wireless communication. It may be understood that the foregoing modem processor may be not integrated into the processor 130.

The I/O subsystem 140 is configured to control an external input/output device, and may include another device input controller 141 and a display controller 142. Optionally, one or more other device input controllers 141 receive a signal from and/or send a signal to the another input device 150. The another input device 150 may include a physical button (a push button, a rocker button, or the like), a dial, a slider switch, a joystick, a click scroll wheel, an optical mouse (the optical mouse is an extension of a touch-sensitive surface that does not display a visual output or an extension of a touch-sensitive surface formed by a touchscreen). It should be noted that the another device input controller 141 may be connected to any one or more of the foregoing devices. The display controller 142 in the I/O subsystem 140 receives a signal from and/or sends a signal to the display 120. After the display 120 detects a user input, the display controller 142 converts the detected user input into an interaction with a user interface object displayed on the display 120, that is, a human-computer interaction is implemented.

The memory 110 may be configured to store a software program and a module, and the processor 130 executes various function applications and processes data of the electronic device 100 by running the software program and module that are stored in the memory 110. The memory 110 may mainly include a program storage area and a data storage area. The program storage area may store an operating system, an application needed by at least one function (such as a sound playback function and an image playback function), and the like. The data storage area may store data (such as audio data and a phone book) created based on use of the electronic device 100 and the like. In addition, the memory 110 may include a high-speed random access memory, or may include a nonvolatile memory, such as at least one magnetic disk storage device, a flash memory device, or another volatile solid-state storage device.

The another input device 150 may be configured to receive digital information or character information that is input, and generate a key signal input related to user settings and function control of the electronic device 100. Specifically, the another input device 150 may include, but is not limited to, one or more of a physical keyboard, a function key (for example, a volume control key or a switch key), a trackball, a mouse, a joystick, and an optical mouse (the optical mouse is an extension of a touch-sensitive surface that does not display a visual output or an extension of a touch-sensitive surface formed by a touchscreen). The another input device 150 is connected to the another device input controller 141 of the I/O subsystem 140, and performs a signal interaction with the processor 130 under the control of the another device input controller 141.

The camera 160 is configured to capture a static image or a video. An optical image of an object is generated through the lens, and is projected onto the photosensitive element. The photosensitive element may be a charge coupled device (charge coupled device, CCD) or a complementary metal-oxide-semiconductor (complementary metal-oxide-semiconductor, CMOS) photoelectric transistor. The photosensitive element converts an optical signal into an electrical signal, and then transfers the electrical signal to an image signal processor (image signal processor, ISP). The image signal processor converts the electrical signal into a digital image signal. The ISP outputs the digital image signal to a digital signal processor (digital signal processor, DSP) for processing. The DSP converts the digital image signal into an image signal in a standard format, for example, RGB or YUV. In some embodiments, the electronic device 100 may include one or N camera 160, where N is a positive integer greater than 1.

The infrared sensor 170 is configured to measure a temperature of a surface of a target object. The infrared sensor 170 may include an optical system, a detection element, and a conversion circuit. The optical system may include a transmission type and a reflection type, and the detection element may include a thermosensitive detection element and a photoelectric detection element. Usually, the thermosensitive detection element may be a thermistor. When the thermistor is exposed to infrared radiation, a temperature rises, resistance changes, and an electrical signal obtained through conversion also changes. Usually, the photoelectric detection element may be a photosensitive element.

The distance sensor 180 is configured to measure a distance between the electronic device 100 and the target object. The distance sensor 180 may be an ultrasonic distance sensor or an infrared distance sensor. This is not limited in this embodiment of this disclosure. The distance measured by the distance sensor 180 may be used to assist the infrared sensor 170 in measuring the temperature of the target object.

The barometric pressure sensor 190 is configured to measure a current barometric pressure value. The barometric pressure value obtained by the barometric pressure sensor 190 through measurement may be used to calibrate a temperature value obtained by the infrared sensor 170 through measurement.

The motor 1A0 may generate a vibration prompt. The motor 1A0 may be configured to provide a vibration feedback after temperature measurement is completed, or may be configured to provide a touch vibration feedback. For example, touch operations performed on different applications (for example, photographing and audio playing) may correspond to different vibration feedback effects. For touch operations performed on different areas of the display 120, the motor 1A0 may also correspond to different vibration feedback effects. Different application scenarios (for example, time reminding, information receiving, an alarm clock, and a game) may also correspond to different vibration feedback effects. A touch vibration feedback effect may be further customized.

The body temperature measurement method provided in an embodiment of this disclosure is now described with reference to FIG. 2 to FIG. 5. The method may be applied to an electronic device 100, and includes the following steps.

Step 101: Determine a current temperature measurement mode in response to a detected first operation for selecting a temperature measurement mode, and recognize a target object to obtain a first feature of the target object.

Specifically, the target object may be a person, an animal, or an object. During specific implementation, the electronic device 100 may display a current operation interface to a user by using a display of the electronic device 100 based on a detected instruction that is input by the user for selecting a temperature measurement mode. During specific implementation, the user may click an icon of the temperature measurement application program in the display of the electronic device 100, to start the temperature measurement application program.

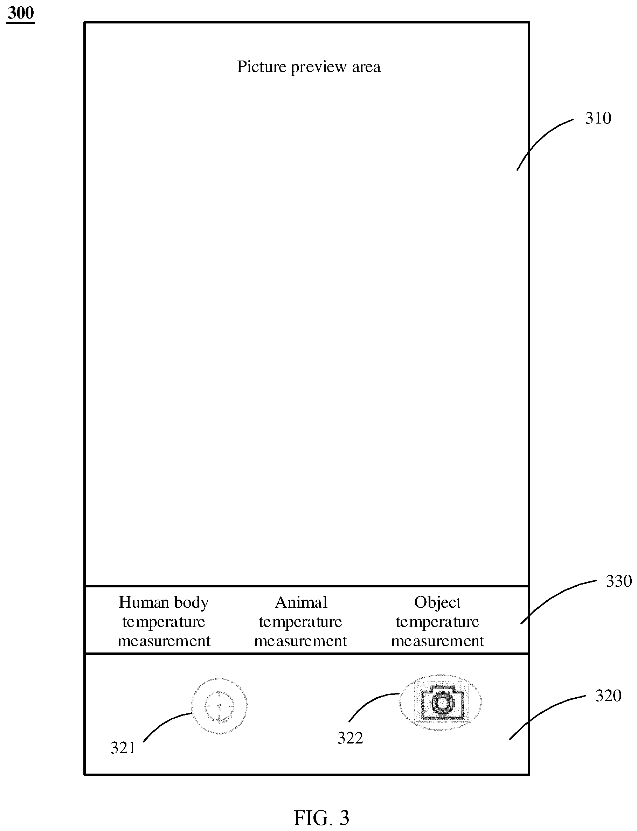

Further, after the user starts the temperature measurement application program, a display interface 300 shown in FIG. 3 may be obtained. With reference to FIG. 3, the display interface 300 of the electronic device 100 may include a picture preview area 310, an operation area 320, and a temperature measurement mode selection area 330. The picture preview area 310 may be configured to display a preview picture obtained by a camera. The operation area 320 includes a temperature measurement button 321 and a camera switching button 322. It should be noted that an electronic device 100 with a single camera has no camera switching function, and therefore, has no camera switching button 322. When the electronic device 100 includes a plurality of cameras and the cameras are located on two sides of the electronic device, the electronic device 100 has a camera switching function, and may include a camera switching button 322. The temperature measurement mode selection area 330 may be configured to select the current temperature measurement mode. Temperature measurement mode options may include human body temperature measurement, animal temperature measurement, and object temperature measurement.

It should be noted that the camera may be turned on after the user starts the temperature measurement application program or after the user determines the temperature measurement mode. For example, the user may click any option in the temperature measurement mode selection area, to determine the current temperature measurement mode, and may turn on the camera to obtain the preview picture. This is not limited in this embodiment of this disclosure.

It can be understood that, the user may alternatively start the temperature measurement application program in another manner, for example, by double-clicking or in another way. This is not limited in this embodiment of this disclosure.

Further, after the user determines the current temperature measurement mode, the electronic device 100 may recognize a preview picture of the target object in the display interface, to obtain the first feature of the target object. The first feature may include a specie of an animal, an age group of a human body, or a part of a human body.

Optionally, if the current temperature measurement mode is a temperature measurement mode for a human body, age and a body part of the target object in the preview picture may be further recognized. To recognize the age of the target object, a plurality of age groups may be distinguished between. For example, age groups of 0 months to 3 months, 3 months to 36 months, and 36 months and above may be obtained through pre-division. When the age of the target object is to be recognized, values in aspects of wrinkles, pores, speckles, elasticity, smoothness, hydration, and the like of skin of the target object may be horizontally compared with pre-collected big data of humans at a same age, with same gender, and in a same race, so that which age group the target object is in can be determined. Age recognition may be specifically performed by using an algorithm in the technical field of artificial intelligence. This is no longer described herein in this disclosure. However, body parts of the target object may include the forehead, a wrist, the neck, and the like. When the body parts of the target object are to be recognized, because skin of each part of a human body is evidently different, recognition may be performed according to a skin texture. For example, there is hair on the scalp, there is fine hair on the body, or a texture on a back of a finger is rough. A macro shot of skin is taken by using a camera, so that skin of different parts may be distinguished between. Therefore, a body part corresponding to the skin can be obtained.

Optionally, if the current temperature measurement mode is a temperature measurement mode for an animal, a specie of the animal may be further recognized. For example, the specie of the animal may include a pig, a dog, a cat, a sheep, cattle, and the like. An image recognition algorithm may use the algorithm in the current technical field of artificial intelligence, and is no longer described herein in this disclosure.

Step 102: Measure a temperature of the target object in response to a detected second operation for measuring the temperature of the target object, to obtain a first temperature value.

Specifically, the electronic device 100 may start temperature measurement on the target object according to a detected temperature measurement instruction that is input by the user. During specific implementation, the user may start body temperature measurement on the target object by clicking a temperature measurement button 311 in the display interface 300. The body temperature of the target object may be measured by using an infrared sensor 170 in the electronic device 100. In this way, a temperature value of the target object, namely, the first temperature value, can be obtained through measurement.

Optionally, before step 102, in other words, before the user measures the temperature of the target object, a distance from the electronic device 100 to the target object may be further measured. The infrared sensor 170 has an optimum measurement distance when a body temperature is measured, and a relatively accurate measurement result may be obtained approximately at the optimum measurement distance. Therefore, a distance threshold may be preset according to the optimum measurement distance. The distance threshold may be a value or a value range. This is not limited in this embodiment of this disclosure. The distance threshold may be related to an application scenario of the infrared sensor 170. For example, when short-distance temperature measurement is performed by using the infrared sensor 170, the distance threshold may be set to 5 cm; or when long-distance temperature measurement is performed by using the infrared sensor 170, the distance threshold may be set to 50 cm.

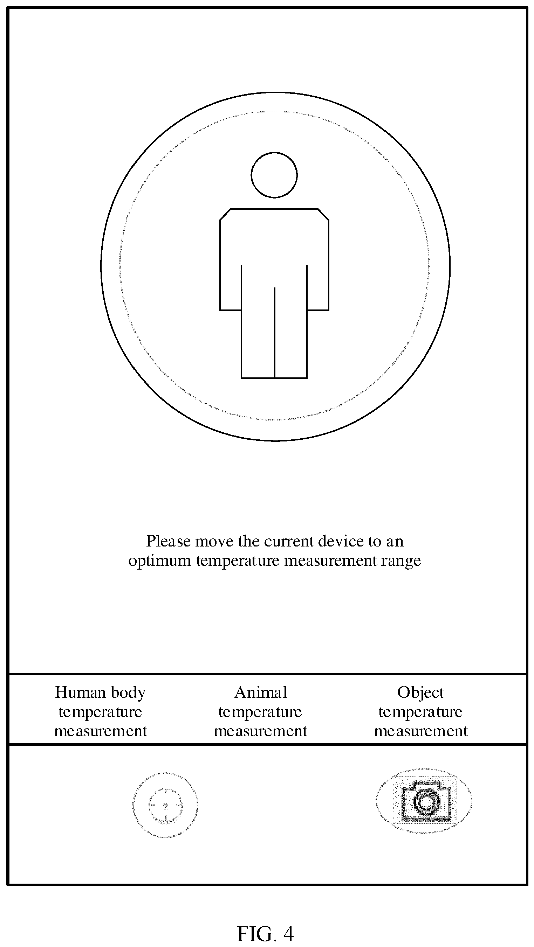

When the distance from the electronic device 100 to the target object is to be measured, measurement may be performed by using a distance sensor 180. A first distance from the electronic device 100 to the target object may be obtained in real time by using the distance sensor 180. After obtaining the first distance, the electronic device 100 may compare the first distance with the preset distance threshold, to determine whether the first distance matches the distance threshold. A matching process may include: If the distance threshold is a value, it may be determined whether the first distance is the same as the distance threshold; or if the distance threshold is a range, it may be determined whether the first distance falls within the range, for example, whether the first distance is greater than an upper limit of the range or less than a lower limit of the range. If the first distance does not match the distance threshold, a prompt may be provided for the user through the display interface 300, and the prompt may be in a form of words or in another form, for example, may be "Please move the current device to an optimum temperature measurement range". A display effect is shown in FIG. 4. Further, if the first distance is greater than the distance threshold, the electronic device 100 may prompt the user to move the electronic device 100 closer, to reduce the distance between the electronic device 100 and the target object. If the first distance is less than the distance threshold, the electronic device 100 may prompt the user to move the electronic device 100 farther, to increase the distance between the electronic device 100 and the target object. If the first distance matches the distance threshold, a prompt may be provided for the user through the display interface 300, and the prompt may be in a form of words or in another form, for example, may be "It is currently in an optimum temperature measurement position, and please measure the temperature".

Further, after the user inputs the temperature measurement instruction, the electronic device 100 starts temperature measurement. At this time, the infrared sensor 170 starts to work. That is, the infrared sensor 170 starts to measure a temperature of a surface of the target object. During temperature measurement, it is necessary to keep the electronic device 100 stable and aim the electronic device 100 at a same part, so that a temperature value obtained through measurement can be accurate. Therefore, a prompt may be provided on the display interface of the electronic device 100. The prompt may be in a form of words or in another form, for example, may be "Please keep the device stable".

Optionally, when the infrared sensor 170 completes temperature measurement and obtains a measured temperature value, namely, the first temperature value, a prompt may be provided on the display interface of the electronic device 100. The prompt may be in a form of words or in another form, for example, may be "The temperature measurement is completed. Please wait". Alternatively, completion of temperature measurement may be prompted through vibration of a motor 1A0 in the electronic device 100. In this case, the user may not need to keep the device stable and may return to a normal state.

Step 103: Obtain a second feature, and compensate for the first temperature value based on the second feature, to obtain a second temperature value; and determine, based on the first feature, whether the second temperature value is normal, and display the second temperature value and a prompt indicating whether the second temperature value is normal.

Specifically, a body temperature of a human body is changeable. For example, the body temperature of the human body may normally fluctuate with changes in factors such as age, day and night, gender, and a mood. For a normal person, a body temperature is the lowest at 2:00 to 6:00 in the morning, and the body temperature is the highest at 2:00 to 8:00 in the afternoon. However, a fluctuation range is from an average value minus 0.5 to the average value plus 0.5. In addition, a body temperature regulation function of a newborn baby is not perfect, and a body temperature is easily affected by an ambient temperature. Due to an increased metabolic rate, a body temperature of a child can be slightly higher than that is an adult. Due to a low metabolic rate, a body temperature of an old person is usually a low value within a normal range. Therefore, the first temperature value that is currently obtained through measurement may be compensated for by using the second feature, to obtain the second temperature value.

The second feature may include one or more of a current system time, a current geographic location, and a current atmospheric environment. During specific implementation, a compensation relationship between the system time and the first temperature value, between the geographic location and the first temperature value, and between the atmospheric environment and the first temperature value may be separately preset. For example, the system time is used as an example. A compensation value of 0.3 degree centigrade may be added to a temperature measurement value at 2 o'clock in the morning, and therefore, a second temperature value may be obtained. However, the compensation value of 0.3 degree centigrade may be subtracted from a temperature measurement value at 2 o'clock in the afternoon, and therefore, a second temperature value may be obtained. The geographic location may be obtained by using a positioning system (Global Positioning System, GPS). According to the claims, the atmospheric environment is represented by using a barometric pressure value, and the barometric pressure value is obtained by using a barometric pressure sensor 190.

Further, after the second temperature value is obtained, the second temperature value may be further displayed on a display 120 of the electronic device 100.

In addition, body temperature values of a human body and an animal usually have normal body temperature ranges. Therefore, when the second temperature value is displayed, a prompt may be further provided for the user to indicate whether the second temperature value is normal.

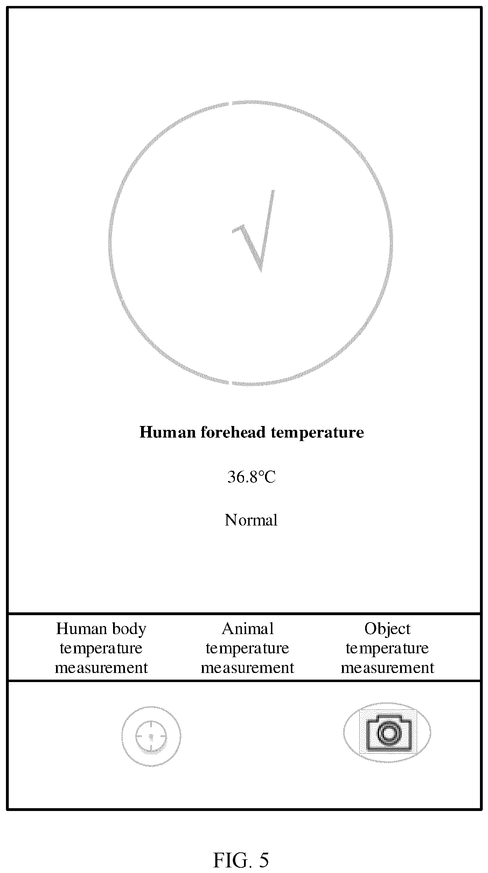

Descriptions are now provided with reference to FIG. 5. First, the normal body temperature ranges of the human body and the animal may be preset in the electronic device 100. Table 1 shows normal body temperature ranges of animals.

**Table 1**

| Animal | Average body temperature value (°C) | Normal body temperature range (°C) |
|---|---|---|
| Buffalo | 38.6 | 37.6 to 39.5 |
| Cattle | 38.2 | 37.9 to 38.6 |
| Goat | 39.1 | 38.5 to 39.7 |
| Pig | 39.2 | 38.7 to 39.8 |
| Dog | 38.9 | 37.9 to 39.9 |
| Cat | 38.6 | 38.1 to 39.2 |
| Chicken | 41.6 | 39.6 to 43.6 |
| Rabbit | 39.5 | 38.6 to 40.1 |

Table 2 shows normal body temperature ranges of age groups of a human body.

**Table 2**

| Age group | Normal body temperature range (°C) |
|---|---|
| 0 years old to 2 years old | 36.4 to 38 |
| 3 years old to 10 years old | 36.1 to 37.8 |
| 11 years old to 65 years old | 35.9 to 37.5 |
| 65 years old and above | 35.8 to 37.4 |

Table 3 shows normal body temperature ranges of body parts of a human body.

**Table 3**

| Body part | Normal body temperature range (°C) |
|---|---|
| Ear temperature | 35.8 to 38 |
| Oral temperature | 35.5 to 37.8 |
| Axillary temperature | 34.7 to 37.2 |
| Rectal temperature | 36.8 to 38 |
| Forehead temperature | 34.6 to 37 |

Then querying may be performed in the foregoing Table 1 to Table 3 based on the first feature obtained in step 101, namely, a specie of an animal, an age group of a human body, and/or a body part of a human body, to obtain the normal body temperature range corresponding to the second temperature value. An example in which a current target object is the human body is used. It is assumed that a forehead temperature is measured and the second temperature value is 36.8°C. It may be obtained by querying Table 3 that the normal body temperature range of the forehead temperature is 34.6°C to 37°C. In this case, the second temperature value falls within the normal body temperature range. That the current body temperature is normal may be prompted, and a display interface is shown in FIG. 5.

This disclosure further provides a body temperature measurement method according to another embodiment, including the following steps.

Step 201: Determine a current temperature measurement mode in response to a detected first operation for selecting a temperature measurement mode, and recognize a target object to obtain a first feature of the target object.

Specifically, a difference between step 201 and step 101 is as follows: If a user selects a temperature measurement mode for a human body, in addition to recognizing an age group and a body part of a current target object, facial recognition may be further performed on the target object. For example, when the user measures a forehead temperature of the target object, facial recognition may be simultaneously performed on the target object. The facial recognition algorithm may use an algorithm in the technical field of artificial intelligence, and is no longer described herein in this disclosure.

An identity of the target object may be recognized through facial recognition, and historical temperature data corresponding to the target object may be obtained according to the identity of the target object. The historical temperature data of the target object may be pre-stored in an electronic device 100, and may be associated with the identity of the target object.

Step 202: Measure a temperature of the target object in response to a detected second operation for measuring the temperature of the target object, to obtain a first temperature value.

Specifically, step 202 is the same as step 102. Details are not described herein again.

Step 203: Obtain a second feature, compensate for the first temperature value based on the second feature, to obtain a second temperature value, and display the second temperature value.

Specifically, a difference between step 203 and step 103 is as follows: After the second temperature value is obtained, the second temperature value may be further stored. In a storage process, the second temperature value may be associated with the identity of the target object. Therefore, when whether the second temperature value is normal is determined, determining may be performed by obtaining the historical temperature data of the target object. The historical temperature data may include temperature data obtained by the electronic device 100 through measurement and temperature data that is input by the user in advance. During specific implementation, the identity of the target object may be obtained through facial recognition in step 201. Then querying may be performed in the electronic device 100 according to the identity of the target object, to obtain the historical temperature data corresponding to the target object. Next, the second temperature value may be compared with the historical temperature data. If the second temperature value matches the historical temperature data, it may be considered that the second temperature value is normal. For example, a process of performing matching between the second temperature value and the historical temperature data may include: obtaining an average value of the historical temperature data. Data within a range from the average value minus 0.5°C to the average value plus 0.5°C may be considered as a normal body temperature range. Alternatively, an interval between a lowest value and a highest value of the historical temperature data may be used as a normal body temperature range. This is not limited in this embodiment of this disclosure. Because body temperature values of some people may not belong to the normal body temperature range, measurement data may be calibrated based on the historical temperature data, so that falsely determining the measurement data can be avoided.

Optionally, after the second temperature value is obtained, the second temperature value may be alternatively displayed on a photo. The second temperature value may be associated with a label attribute of the captured photo. For example, a photo may be associated with a plurality of label attributes, such as a date, a location, and a body temperature. Alternatively, the second temperature value may be directly displayed on a captured photo. This is not limited in this embodiment of this disclosure.

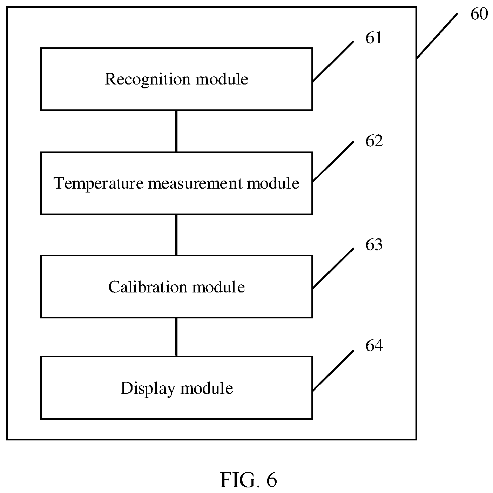

FIG. 6 is a schematic diagram of a structure of an embodiment of a body temperature measurement apparatus according to this disclosure. As shown in FIG. 6, the body temperature measurement apparatus 60 may include: a recognition module 61, a temperature measurement module 62, a calibration module 63, and a display module 64.

The recognition module 61 is configured to detect a first operation for selecting a temperature measurement mode, determine a current temperature measurement mode in response to the detected first operation, and recognize a target object in the current temperature measurement mode to obtain a first feature of the target object.

The temperature measurement module 62 is configured to detect a second operation for measuring a temperature of the target object, and measure the temperature of the target object in response to the detected second operation, to obtain a first temperature value.

The calibration module 63 is configured to obtain a second feature, and compensate for the first temperature value based on the second feature, to obtain a second temperature value.

The display module 64 is configured to determine, based on the first feature, whether the second temperature value is normal, and display the second temperature value and a prompt indicating whether the second temperature value is normal.

In a possible implementation, the apparatus 60 may further include a distance measurement module 65.

The distance measurement module 65 is configured to: obtain a first distance to the target object, and compare the first distance with a preset distance threshold; and if the first distance does not match the preset distance threshold, display a first prompt; or if the first distance matches the preset distance threshold, display a second prompt.

In a possible implementation, the apparatus 60 may further include a storage module 66.

The storage module 66 is configured to associate the second temperature value with an identity of the target object, and store the second temperature value.

In a possible implementation, the apparatus 60 may further include a facial recognition module 67.

The facial recognition module 67 is configured to: perform facial recognition on the target object, to obtain the identity of the target object; perform querying according to the identity of the target object, to obtain a historical temperature value of the target object; and compare the second temperature value with the historical temperature value, to determine, based on a comparison result, whether the second temperature value is normal.

In a possible implementation, the display module 64 may include a querying unit 641 and a comparison unit 642.

The querying unit 641 is configured to perform querying based on the first feature, to obtain a preset temperature threshold corresponding to the first feature.

The comparison unit 642 is configured to compare the second temperature value with the preset temperature threshold, and determine, based on a comparison result, whether the second temperature value is normal.

In a possible implementation, the apparatus 60 may further include an association module 68.

The association module 68 is configured to obtain a first image, and display the second temperature value on the first image.

It should be understood that division of the modules of the body temperature measurement apparatus shown in FIG. 6 above is only division of logical functions. During actual implementation, the modules may be all or partially integrated into one physical entity, or may be physically separated. In addition, the modules may be all implemented in a form of software invoking processing elements; or the modules may be all implemented in a form of hardware; or some modules may be implemented in a form of software invoking processing elements, and some modules may be implemented in a form of hardware. For example, the detection module may be an independently disposed processing element, or may be integrated in one chip of an electronic device for implementation. Implementation of another module is similar to the implementation of the detection module. In addition, all or some of the modules may be integrated together, or may be independently implemented. In an implementation process, steps in the foregoing methods or the foregoing modules can be implemented by using a hardware integrated logical circuit in the processing element, or by using instructions in a form of software.

For example, the foregoing modules may be one or more integrated circuits configured to implement the foregoing method, such as one or more application specific integrated circuits (Application Specific Integrated Circuit, ASIC for short below), one or more digital signal processors (Digital Signal Processor, DSP for short below), or one or more field programmable gate arrays (Field Programmable Gate Array, FPGAfor short below). For another example, the modules may be integrated together and implemented in a form of a system-on-a-chip (System-On-a-Chip, SOC for short below).

It may be understood that an interface connection relationship between the modules that is shown in this embodiment of the present disclosure is merely an example for description, and does not constitute a limitation on a structure of the electronic device 100. In some other embodiments of this disclosure, the electronic device 100 may alternatively use an interface connection manner different from that in the foregoing embodiment, or use a combination of a plurality of interface connection manners.

It may be understood that, to implement the foregoing functions, the electronic device and the like include a hardware structure and/or a software module corresponding to each function. A person skilled in the art should be easily aware that, in combination with the examples described in embodiments disclosed in this specification, units, algorithms, and steps may be implemented by hardware or a combination of hardware and computer software in embodiments of this disclosure. Whether a function is performed by hardware or hardware driven by computer software depends on a particular application and a design constraint of the technical solutions. A person skilled in the art may use different methods to implement the described functions for each particular application, but it should not be considered that the implementation goes beyond the scope of embodiments of the present disclosure.

In this embodiment of this disclosure, the electronic device and the like may be divided into functional modules according to the foregoing method examples. For example, each functional module may be correspondingly obtained through division based on each function, or two or more functions may be integrated into one processing module. The integrated module may be implemented in a form of hardware, or may be implemented in a form of a software functional module. It should be noted that, in embodiments of the present disclosure, division into the modules is an example and is merely logical function division, and may be other division in an actual implementation.

Based on the foregoing descriptions of the implementations, a person skilled in the art may clearly understand that for the purpose of convenient and brief descriptions, division into the foregoing functional modules is merely used as an example for descriptions. During actual application, the foregoing functions can be allocated to different functional modules for implementation based on a requirement, in other words, an inner structure of an apparatus is divided into different functional modules to implement all or a part of the functions described above. For a specific working process of the foregoing system, apparatus, and unit, refer to a corresponding process in the foregoing method embodiments, and details are not described herein again.

Functional units in embodiments of this disclosure may be integrated into one processing unit, or each of the units may exist alone physically, or two or more units are integrated into one unit. The integrated unit may be implemented in a form of hardware, or may be implemented in a form of a software function unit.

When the integrated unit is implemented in the form of a software functional unit and sold or used as an independent product, the integrated unit may be stored in a computer-readable storage medium. Based on such an understanding, the technical solutions of embodiments of this disclosure essentially, or the part contributing to the conventional technology, or all or some of the technical solutions may be implemented in the form of a software product. The computer software product is stored in a storage medium and includes several instructions for instructing a computer device (which may be a personal computer, a server, a network device, or the like) or the processor 130 to perform all or some of the steps of the methods described in embodiments of this disclosure. The foregoing storage medium includes any medium that can store program code, for example, a flash memory, a removable hard disk, a read-only memory, a random access memory, a magnetic disk, or an optical disc.

The foregoing descriptions are only specific implementations of this disclosure, but are not intended to limit the protection scope of this disclosure. Any variation or replacement within the technical scope disclosed in this disclosure shall fall within the protection scope of this disclosure. Therefore, the protection scope of this disclosure shall be subject to the protection scope of the claims.

## Claims

1. A body temperature measurement method, wherein the method comprises performing the following steps by an electronic device (100):
detecting a first operation for selecting a temperature measurement mode;
determining (101) a current temperature measurement mode in response to the detected first operation, obtain a preview picture of a target object using a camera, and recognizing the preview picture of a target object in the current temperature measurement mode to obtain a first feature of the target object;
detecting a second operation for measuring a temperature of the target object;
measuring (102), using an infrared sensor (170), the temperature of the target object in response to the detected second operation, to obtain a first temperature value;
obtaining (103) a second feature, and compensating for the first temperature value based on the second feature, to obtain a second temperature value, wherein the second feature comprises one or more of a current system time, a current geographic location obtained by using a positioning system, and a current atmospheric environment represented by a barometric pressure value obtained by using a barometric pressure sensor (190); and
determining, based on the first feature, whether the second temperature value is normal, by comparing the second temperature value with the normal range corresponding to the first feature, and displaying the second temperature value and a prompt indicating whether the second temperature value is normal.

2. The method according to claim 1, wherein the method further comprises:
obtaining a first distance to the target object, and comparing the first distance with a preset distance threshold; and
if the first distance does not match the preset distance threshold, displaying a first prompt; or
if the first distance matches the preset distance threshold, displaying a second prompt.

3. The method according to claim 1, wherein the method further comprises:
associating the second temperature value with an identity of the target object, and storing the second temperature value.

4. The method according to claim 3, wherein the method further comprises:
performing facial recognition on the target object, to obtain the identity of the target object;
performing querying according to the identity of the target object, to obtain a historical temperature value of the target object; and
comparing the second temperature value with the historical temperature value, to determine, based on a comparison result, whether the second temperature value is normal.

5. The method according to claim 1, wherein the determining, based on the first feature, whether the second temperature value is normal comprises:
performing querying based on the first feature, to obtain a preset temperature threshold corresponding to the first feature; and
comparing the second temperature value with the preset temperature threshold, and determining, based on a comparison result, whether the second temperature value is normal.

6. The method according to claim 1, wherein the method further comprises:
obtaining a first image, and displaying the second temperature value on the first image.

7. An electronic device (100), comprising a camera (160), an infrared sensor (170) and a memory (110), wherein the memory (110) is configured to store computer program code, the computer program code comprises instructions, and when the device (100) reads the instructions from the memory (110), the device (100) is enabled to perform the following steps:
detecting a first operation for selecting a temperature measurement mode;
determining a current temperature measurement mode in response to the detected first operation, obtain a preview picture by using the camera (160), and recognizing the preview picture of a target object in the current temperature measurement mode to obtain a first feature of the target object;
detecting a second operation for measuring a temperature of the target object;
measuring with the infrared sensor (170) temperature of the target object in response to the detected second operation, to obtain a first temperature value;
obtaining a second feature, and compensating for the first temperature value based on the second feature, to obtain a second temperature value, wherein the second feature comprises one or more of a current system time, a current geographic location obtained by using a positioning system, and a current atmospheric environment represented by a barometric pressure value obtained by using a barometric pressure sensor (190); and
determining, based on the first feature, whether the second temperature value is normal, by comparing the second temperature value with the normal range corresponding to the first feature, and displaying the second temperature value and a prompt indicating whether the second temperature value is normal.

8. The device (100) according to claim 7, wherein when the instructions are executed by the device (100), the device (100) is enabled to further perform the following steps:
obtaining a first distance to the target object, and comparing the first distance with a preset distance threshold; and
if the first distance does not match the preset distance threshold, displaying a first prompt; or
if the first distance matches the preset distance threshold, displaying a second prompt.

9. The device (100) according to claim 7, wherein when the instructions are executed by the device (100), the device (100) is enabled to further perform the following steps:
associating the second temperature value with an identity of the target object, and storing the second temperature value.

10. The device (100) according to claim 9, wherein when the instructions are executed by the device (100), the device (100) is enabled to further perform the following steps:
performing facial recognition on the target object, to obtain the identity of the target object;
performing querying according to the identity of the target object, to obtain a historical temperature value of the target object; and
comparing the second temperature value with the historical temperature value, to determine, based on a comparison result, whether the second temperature value is normal.

11. The device (100) according to claim 7, wherein when the instructions are executed by the device (100), enabling the device (100) to perform the step of determining, based on the first feature, whether the second temperature value is normal comprises:
performing querying based on the first feature, to obtain a preset temperature threshold corresponding to the first feature; and
comparing the second temperature value with the preset temperature threshold, and determining, based on a comparison result, whether the second temperature value is normal.

12. The device (100) according to claim 7, wherein when the instructions are executed by the device (100), the device (100) is enabled to further perform the following steps:
obtaining a first image, and displaying the second temperature value on the first image.

13. A computer-readable storage medium, comprising computer instructions, wherein when the computer instructions are run on a device according to claim 7, the device is enabled to perform the method according to any one of claims 1 to 6.

14. A computer program product, wherein when the computer program product is run on the device (100) of claim 7, the device (100) is enabled to perform the method according to any one of claims 1 to 6.

## Patentansprüche

1. Körpertemperaturmessverfahren, wobei das Verfahren ein Durchführen der folgenden Schritte durch eine elektronische Vorrichtung (100) umfasst:
Erfassen einer ersten Operation zum Auswählen eines Temperaturmessmodus;
Bestimmen (101) eines aktuellen Temperaturmessmodus als Reaktion auf die erfasste erste Operation, Erhalten eines Vorschaubilds eines Zielobjekts unter Verwendung einer Kamera,
und Erkennen des Vorschaubilds eines Zielobjekts in dem aktuellen Temperaturmessmodus, um ein erstes Merkmal des Zielobjekts zu erhalten;
Erfassen einer zweiten Operation zum Messen einer Temperatur des Zielobjekts;
Messen (102) der Temperatur des Zielobjekts unter Verwendung eines Infrarotsensors (170) als Reaktion auf die erfasste zweite Operation, um einen ersten Temperaturwert zu erhalten;
Erhalten (103) eines zweiten Merkmals und Kompensieren des ersten Temperaturwerts basierend auf dem zweiten Merkmal, um einen zweiten Temperaturwert zu erhalten, wobei das zweite Merkmal eines oder mehrere von einer aktuellen Systemzeit, einem aktuellen geografischen Standort, der durch Verwenden eines Positionierungssystems erhalten wird, und einer aktuellen atmosphärischen Umgebung, die durch einen Luftdruckwert repräsentiert wird, der durch Verwenden eines Luftdrucksensors (190) erhalten wird, umfasst; und
Bestimmen, basierend auf dem ersten Merkmal, ob der zweite Temperaturwert normal ist, durch Vergleichen des zweiten Temperaturwerts mit dem Normalbereich, der dem ersten Merkmal entspricht, und Anzeigen des zweiten Temperaturwerts und eines Hinweises, der angibt, ob der zweite Temperaturwert normal ist.

2. Verfahren nach Anspruch 1, wobei das Verfahren ferner umfasst:
Erhalten eines ersten Abstands zu dem Zielobjekt und Vergleichen des ersten Abstands mit einem voreingestellten Abstandsschwellenwert; und
falls der erste Abstand nicht mit dem voreingestellten Abstandsschwellenwert übereinstimmt, Anzeigen eines ersten Hinweises; oder
falls der erste Abstand mit dem voreingestellten Abstandsschwellenwert übereinstimmt, Anzeigen eines zweiten Hinweises.

3. Verfahren nach Anspruch 1, wobei das Verfahren ferner umfasst:
Verknüpfen des zweiten Temperaturwerts mit einer Identität des Zielobjekts und Speichern des zweiten Temperaturwerts.

4. Verfahren nach Anspruch 3, wobei das Verfahren ferner umfasst:
Durchführen von Gesichtserkennung an dem Zielobjekt, um die Identität des Zielobjekts zu erhalten;
Durchführen einer Abfrage gemäß der Identität des Zielobjekts, um einen historischen Temperaturwert des Zielobjekts zu erhalten; und
Vergleichen des zweiten Temperaturwerts mit dem historischen Temperaturwert, um basierend auf einem Vergleichsergebnis zu bestimmen, ob der zweite Temperaturwert normal ist.

5. Verfahren nach Anspruch 1, wobei das Bestimmen, basierend auf dem ersten Merkmal, ob der zweite Temperaturwert normal ist, umfasst:
Durchführen einer Abfrage basierend auf dem ersten Merkmal, um einen voreingestellten Temperaturschwellenwert zu erhalten, der dem ersten Merkmal entspricht; und
Vergleichen des zweiten Temperaturwerts mit dem voreingestellten Temperaturschwellenwert und Bestimmen, basierend auf einem Vergleichsergebnis, ob der zweite Temperaturwert normal ist.

6. Verfahren nach Anspruch 1, wobei das Verfahren ferner umfasst:
Erhalten eines ersten Bilds und Anzeigen des zweiten Temperaturwerts auf dem ersten Bild.

7. Elektronische Vorrichtung (100), umfassend eine Kamera (160), einen Infrarotsensor (170) und einen Speicher (110), wobei der Speicher (110) dazu konfiguriert ist, Computerprogrammcode zu speichern, wobei der Computerprogrammcode Befehle umfasst, und wenn die Vorrichtung (100) die Befehle aus dem Speicher (110) liest, es der Vorrichtung (100) ermöglicht wird, die folgenden Schritte durchzuführen:
Erfassen einer ersten Operation zum Auswählen eines Temperaturmessmodus;
Bestimmen eines aktuellen Temperaturmessmodus als Reaktion auf die erfasste erste Operation, Erhalten eines Vorschaubilds durch Verwenden der Kamera (160) und Erkennen des Vorschaubilds eines Zielobjekts in dem aktuellen Temperaturmessmodus, um ein erstes Merkmal des Zielobjekts zu erhalten;
Erfassen einer zweiten Operation zum Messen einer Temperatur des Zielobjekts;
Messen der Temperatur des Zielobjekts mit dem Infrarotsensor (170) als Reaktion auf die erfasste zweite Operation, um einen ersten Temperaturwert zu erhalten;
Erhalten eines zweiten Merkmals und Kompensieren des ersten Temperaturwerts basierend auf dem zweiten Merkmal, um einen zweiten Temperaturwert zu erhalten, wobei das zweite Merkmal eines oder mehrere von einer aktuellen Systemzeit, einem aktuellen geografischen Standort, der durch Verwenden eines Positionierungssystems erhalten wird, und einer aktuellen atmosphärischen Umgebung, die durch einen Luftdruckwert repräsentiert wird, der durch Verwenden eines Luftdrucksensors (190) erhalten wird, umfasst; und
Bestimmen, basierend auf dem ersten Merkmal, ob der zweite Temperaturwert normal ist, durch Vergleichen des zweiten Temperaturwerts mit dem Normalbereich, der dem ersten Merkmal entspricht, und Anzeigen des zweiten Temperaturwerts und eines Hinweises, der angibt, ob der zweite Temperaturwert normal ist.

8. Vorrichtung (100) nach Anspruch 7, wobei, wenn die Befehle durch die Vorrichtung (100) ausgeführt werden, es der Vorrichtung (100) ermöglicht wird, ferner die folgenden Schritte durchzuführen:
Erhalten eines ersten Abstands zu dem Zielobjekt und Vergleichen des ersten Abstands mit einem voreingestellten Abstandsschwellenwert; und
falls der erste Abstand nicht mit dem voreingestellten Abstandsschwellenwert übereinstimmt, Anzeigen eines ersten Hinweises; oder
falls der erste Abstand mit dem voreingestellten Abstandsschwellenwert übereinstimmt, Anzeigen eines zweiten Hinweises.

9. Vorrichtung (100) nach Anspruch 7, wobei, wenn die Befehle durch die Vorrichtung (100) ausgeführt werden, es der Vorrichtung (100) ermöglicht wird, ferner die folgenden Schritte durchzuführen:
Verknüpfen des zweiten Temperaturwerts mit einer Identität des Zielobjekts und Speichern des zweiten Temperaturwerts.

10. Vorrichtung (100) nach Anspruch 9, wobei, wenn die Befehle durch die Vorrichtung (100) ausgeführt werden, es der Vorrichtung (100) ermöglicht wird, ferner die folgenden Schritte durchzuführen:
Durchführen von Gesichtserkennung an dem Zielobjekt, um die Identität des Zielobjekts zu erhalten;
Durchführen einer Abfrage gemäß der Identität des Zielobjekts, um einen historischen Temperaturwert des Zielobjekts zu erhalten; und
Vergleichen des zweiten Temperaturwerts mit dem historischen Temperaturwert, um basierend auf einem Vergleichsergebnis zu bestimmen, ob der zweite Temperaturwert normal ist.

11. Vorrichtung (100) nach Anspruch 7, wobei, wenn die Befehle durch die Vorrichtung (100) ausgeführt werden, das Ermöglichen, dass die Vorrichtung (100) den Schritt des Bestimmens, basierend auf dem ersten Merkmal, ob der zweite Temperaturwert normal ist, durchführt, umfasst:
Durchführen einer Abfrage basierend auf dem ersten Merkmal, um einen voreingestellten Temperaturschwellenwert zu erhalten, der dem ersten Merkmal entspricht; und
Vergleichen des zweiten Temperaturwerts mit dem voreingestellten Temperaturschwellenwert und Bestimmen, basierend auf einem Vergleichsergebnis, ob der zweite Temperaturwert normal ist.

12. Vorrichtung (100) nach Anspruch 7, wobei, wenn die Befehle durch die Vorrichtung (100) ausgeführt werden, es der Vorrichtung (100) ermöglicht wird, ferner die folgenden Schritte durchzuführen:
Erhalten eines ersten Bilds und Anzeigen des zweiten Temperaturwerts auf dem ersten Bild.

13. Computerlesbares Speichermedium, umfassend Computerbefehle, wobei, wenn die Computerbefehle auf einer Vorrichtung nach Anspruch 7 vorgenommen werden, es der Vorrichtung ermöglicht wird, das Verfahren nach einem der Ansprüche 1 bis 6 durchzuführen.

14. Computerprogrammprodukt, wobei, wenn das Computerprogrammprodukt auf der Vorrichtung (100) nach Anspruch 7 vorgenommen wird, es der Vorrichtung (100) ermöglicht wird, das Verfahren nach einem der Ansprüche 1 bis 6 durchzuführen.

## Revendications

1. Procédé de mesure de température corporelle, dans lequel le procédé comprend la réalisation des étapes suivantes par un dispositif électronique (100) :
la détection d'une première opération pour la sélection d'un mode de mesure de température ;
la détermination (101) d'un mode de mesure de température actuel en réponse à la première opération détectée, obtenir une image d'aperçu d'un objet cible à l'aide d'une caméra, et la reconnaissance de l'image d'aperçu d'un objet cible dans le mode de mesure de température actuel pour obtenir une première caractéristique de l'objet cible ;
la détection d'une seconde opération pour mesurer la température de l'objet cible ;
la mesure (102), à l'aide d'un capteur infrarouge (170), de la température de l'objet cible en réponse à la seconde opération détectée, pour obtenir une première valeur de température ;
l'obtention (103) d'une seconde caractéristique, et la compensation de la première valeur de température sur la base de la seconde caractéristique, pour obtenir une seconde valeur de température, dans lequel la seconde caractéristique comprend un ou plusieurs éléments parmi l'heure système actuelle, la position géographique actuelle obtenue à l'aide d'un système de positionnement et l'environnement atmosphérique actuel représenté par une valeur de pression barométrique obtenue à l'aide d'un capteur de pression barométrique (190) ; et
le fait de déterminer, sur la base de la première caractéristique, si la seconde valeur de température est normale, en comparant la seconde valeur de température à la plage normale correspondant à la première caractéristique, et l'affichage de la seconde valeur de température et d'une invite indiquant si la seconde valeur de température est normale.

2. Procédé selon la revendication 1, dans lequel le procédé comprend en outre :
l'obtention d'une première distance à l'objet cible, et la comparaison de la première distance à un seuil de distance prédéfini ; et
si la première distance ne correspond pas au seuil de distance prédéfini, l'affichage d'une première invite ; ou
si la première distance correspond au seuil de distance prédéfini, l'affichage d'une seconde invite.

3. Procédé selon la revendication 1, dans lequel le procédé comprend en outre :
l'association de la seconde valeur de température à une identité de l'objet cible et le stockage de la seconde valeur de température.

4. Procédé selon la revendication 3, dans lequel le procédé comprend en outre :
la réalisation d'une reconnaissance faciale sur l'objet cible, pour obtenir l'identité de l'objet cible ;
la réalisation d'une requête en fonction de l'identité de l'objet cible, pour obtenir une valeur de température historique de l'objet cible ; et
la comparaison de la seconde valeur de température avec la valeur de température historique, pour déterminer, sur la base d'un résultat de comparaison, si la seconde valeur de température est normale.

5. Procédé selon la revendication 1, dans lequel le fait de déterminer, sur la base de la première caractéristique, si la seconde valeur de température est normale comprend :
la réalisation d'une requête sur la base de la première caractéristique, pour obtenir un seuil de température prédéfini correspondant à la première caractéristique ; et
la comparaison de la seconde valeur de température avec le seuil de température prédéfini et le fait de déterminer, sur la base d'un résultat de comparaison, si la seconde valeur de température est normale.

6. Procédé selon la revendication 1, dans lequel le procédé comprend en outre :
l'obtention d'une première image et l'affichage de la seconde valeur de température sur la première image.

7. Dispositif électronique (100) comprenant une caméra (160), un capteur infrarouge (170) et une mémoire (110), dans lequel la mémoire (110) est configurée pour stocker un code de programme informatique, le code de programme informatique comprend des instructions, et lorsque le dispositif (100) lit les instructions à partir de la mémoire (110), le dispositif (100) est activé pour réaliser les étapes suivantes :
la détection d'une première opération pour la sélection d'un mode de mesure de température ;
la détermination d'un mode de mesure de température actuel en réponse à la première opération détectée, obtenir une image d'aperçu à l'aide de la caméra (160) et la reconnaissance de l'image d'aperçu d'un objet cible dans le mode de mesure de température actuel pour obtenir une première caractéristique de l'objet cible ;
la détection d'une seconde opération pour mesurer la température de l'objet cible ;
la mesure avec le capteur infrarouge (170) de la température de l'objet cible en réponse à la seconde opération détectée, pour obtenir une première valeur de température ;
l'obtention d'une seconde caractéristique, et la compensation de la première valeur de température sur la base de la seconde caractéristique, pour obtenir une seconde valeur de température, dans lequel la seconde caractéristique comprend un ou plusieurs éléments parmi l'heure système actuelle, la position géographique actuelle obtenue à l'aide d'un système de positionnement et l'environnement atmosphérique actuel représenté par une valeur de pression barométrique obtenue à l'aide d'un capteur de pression barométrique (190) ; et
le fait de déterminer, sur la base de la première caractéristique, si la seconde valeur de température est normale, en comparant la seconde valeur de température à la plage normale correspondant à la première caractéristique, et l'affichage de la seconde valeur de température et d'une invite indiquant si la seconde valeur de température est normale.

8. Dispositif (100) selon la revendication 7, dans lequel, lorsque les instructions sont exécutées par le dispositif (100), le dispositif (100) est activé pour en outre réaliser les étapes suivantes :
l'obtention d'une première distance à l'objet cible, et la comparaison de la première distance à un seuil de distance prédéfini ; et
si la première distance ne correspond pas au seuil de distance prédéfini, l'affichage d'une première invite ; ou
si la première distance correspond au seuil de distance prédéfini, l'affichage d'une seconde invite.

9. Dispositif (100) selon la revendication 7, dans lequel, lorsque les instructions sont exécutées par le dispositif (100), le dispositif (100) est activé pour en outre réaliser les étapes suivantes :
l'association de la seconde valeur de température à une identité de l'objet cible et le stockage de la seconde valeur de température.

10. Dispositif (100) selon la revendication 9, dans lequel, lorsque les instructions sont exécutées par le dispositif (100), le dispositif (100) est activé pour en outre réaliser les étapes suivantes :
la réalisation d'une reconnaissance faciale sur l'objet cible, pour obtenir l'identité de l'objet cible ;
la réalisation d'une requête en fonction de l'identité de l'objet cible, pour obtenir une valeur de température historique de l'objet cible ; et
la comparaison de la seconde valeur de température avec la valeur de température historique, pour déterminer, sur la base d'un résultat de comparaison, si la seconde valeur de température est normale.

11. Dispositif (100) selon la revendication 7, dans lequel, lorsque les instructions sont exécutées par le dispositif (100), le dispositif (100) est activé pour réaliser l'étape du fait de déterminer, sur la base de la première caractéristique, si la seconde valeur de température est normale, comprend :
la réalisation d'une requête sur la base de la première caractéristique, pour obtenir un seuil de température prédéfini correspondant à la première caractéristique ; et
la comparaison de la seconde valeur de température avec le seuil de température prédéfini et le fait de déterminer, sur la base d'un résultat de comparaison, si la seconde valeur de température est normale.

12. Dispositif (100) selon la revendication 7, dans lequel, lorsque les instructions sont exécutées par le dispositif (100), le dispositif (100) est activé pour en outre réaliser les étapes suivantes :
l'obtention d'une première image et l'affichage de la seconde valeur de température sur la première image.

13. Support d'enregistrement lisible par ordinateur, comprenant des instructions informatiques, dans lequel, lorsque les instructions informatiques sont exécutées sur un dispositif selon la revendication 7, le dispositif est activé pour réaliser le procédé selon l'une quelconque des revendications 1 à 6.

14. Produit de programme informatique, dans lequel, lorsque le produit de programme informatique est exécuté sur le dispositif (100) selon la revendication 7, le dispositif (100) est activé pour réaliser le procédé selon l'une quelconque des revendications 1 à 6.
